# EUROPEAN PATENT APPLICATION

(11) **EP 3 078 657 A1**
(43) Date of publication of application: **12.10.2016**
(21) Application number: 15162887.2
(22) Date of filing: 09.04.2015
(51) Int. Cl.: C07C 409/16, H01B 3/56

(54) **Compounds for dielectrically insulating electric active parts**

(71) Applicant: Solvay SA, 1120 Bruxelles (BE)
(72) Inventor: The designation of the inventor has not yet been filed
(74) Representative: Mross, Stefan P.M.

(57) **Abstract**

The invention concerns compounds, gas mixtures as well as methods for dielectrically insulating electric active parts using certain fluorinated peroxide compounds.

## Description

The invention concerns compounds, gas mixtures as well as methods for dielectrically insulating electric active parts using certain fluorinated peroxide compounds.

Dielectric insulation media in liquid or gaseous state are applied for the insulation of electrical active parts in a wide variety of electrical apparatuses, e.g. in switchgears or transformers.

Mixtures of SF₆ and N₂ are widely applied as dielectric insulating gas. Efforts have been made in the past to provide alternative dielectric insulating gases.

WO 2014/096414 concerns a method of dielectrically insulating electric active parts using certain fluorinated compounds, e.g. fluorinated peroxides, and discloses CF₃-O-O-CF₃ specifically.

The object of the present invention is to provide improved compounds, gas mixtures and methods for the electrical insulation of electrical active parts.

Advantageously, the compounds and gas mixtures of the present invention show improved insulation, arc-extinguishing and/or switching performance. Also advantageously, the compounds and gas mixtures of the present invention show advantageous environmental impact when released into the atmosphere, e.g. as measured by an improved global warming potential (GWP) and/or improved ozone depletion. Also advantageously, the compounds and gas mixtures of the present invention show an improved toxicological behavior, as measured for example by a higher LC50 and/or a higher Occupational Exposure Limit. Furthermore, the compounds and gas mixtures advantageously show an improved dew point, vapour pressure, boiling point, dielectric strengths, and/or thermal stability. Additionally, the compounds according to this invention advantageously show an improved chemical inertness against the construction materials used e.g. for the electric active parts and/or improved heat transfer properties. Also advantageously, the inventive compounds can be readily prepared with low cost.

These and other objectives are solved by the present invention as outlined in the claims.

Accordingly, a first aspect of the present invention concerns a compound of the general formula R1-O-O-R2 wherein R1 and R2 are independently chosen from the group consisting of perfluorinated or partially fluorinated methyl, ethyl, isopropyl, n-propyl, isobutyl, n-butyl or tert-butyl, with the proviso that if R1 is CF₃, R2 is not CF₃.

Thus, compounds that contain independently from 1 to 4 carbon atoms in R1 as well as in R2 can be suitably used.

Preferably, R1 and R2 are perfluorinated, i.e. all hydrogen atoms in the alkyl groups R1 and R2 have been replaced by fluorine atoms. Thus, R1 and R2 can independently be chosen from the group consisting of perfluorinated methyl, ethyl, isopropyl, n-propyl, isobutyl, n-butyl or tert-butyl groups, more preferably, chosen from trifluoromethyl, pentafluoroethyl, and heptafluoroisopropyl.

Alternatively, R1 and/or R2 are not perfluorinated. In this case, R1 and R2 are independently chosen from the group consisting of partially fluorinated methyl, ethyl, isopropyl, n-propyl, isobutyl, n-butyl and or tert-butyl. Preferably, R1 and R2 are indepedently chosen from difluoromethyl, tetrafluoroethyl, n-hexafluoropropyl and isohexafluoropropyl, more preferably difluoromethyl.

R1 and R2 can preferably be the same. In an alternative preferred embodiment, R1 and R2 are different.

Specifically, the compounds of the present invention are CF₃CF₂-O-O-CF₂CF₃, (CF₃)₂CF-O-O-CF(CF₃)₂, CF₃-O-O-CF₂CF₃, and CF₃-O-O-CF(CF₃)₂, CF₃CF₂-O-O-CF(CF₃)₂

Preferably, the compound according to the invention has a standard boiling point, i.e. a boiling point at a pressure of 1 bar, of less than 20 °C, more preferably equal to or lower than 0 °C, even more preferably equal to or less than -10°C. Also preferably, the compound according to the invention has a standard boiling point of equal to or higher than -80°C, more preferably equal to or higher than -50°C.

In the frame of the present invention, the singular is intended to include the plural, and vice versa.

A second aspect of the invention concerns a gas mixture of two or more compounds of the general formula R1-O-O-R2 wherein R1 and R2 are independently chosen from the group consisting of perfluorinated or partially fluorinated methyl, ethyl, isopropyl, n-propyl, isobutyl, n-butyl or tert-butyl. Preferably, the two or more compounds are chosen from the group consisting of CF₃-O-O-CF₃, CF₃CF₂-O-O-CF₂CF₃, (CF₃)₂CF-O-O-CF(CF₃)₂, CF₃-O-O-CF₂CF₃, or CF₃-O-O-CF(CF₃)₂.

The gas mixture according to this invention can advantageously lead to an improvement of at least one of the advantages as described above. Compounds used as a dielectric are used to prevent and/or quickly quench electric discharges. However, these compounds often undergo cleavage by breakage of certain chemical bonds in the chemical structure leading to the formation of radical and/or ions. Without being bond to a theory, the inventors believe that the compounds and especially the gas mixtures according to this invention have the advantage that the recombination of e.g. the possible radicals formed in such cleavage, leads to the same mixture of compound or to a mixture with similar properties. By way of example, a possible radical cleavage of a mixture of CF₃-O-O-CF₃ and CF₃CF₂-O-O-CF₂CF₃, may lead to the advantageous mixture of CF₃-O-O-CF₃, CF₃CF₂-O-O-CF₂CF₃ and CF₃CF₂-O-O-CF₃.

Preferably, the inventive gas mixtures further comprise at least one compound selected from an inert gas, a perfluorinated or partially fluorinated ketone, a perfluorinated or partially fluorinated ether, a perfluorinated or partially fluorinated ester, a perfluorinated or partially fluorinated cyano compound, a hydrocarbon compound, more preferably an inert gas.

The term "inert gas" is intended to denote a gas that does not react with the compounds according to the invention. Preferably, the inert gas is chosen from the list consisting of air, synthetic air, an air component, N₂, O₂, CO₂, N₂O, He, Ne, Ar, Xe or SF₆; more preferably, the inert gas is N₂.

Preferably, the at least one compound is a perfluorinated or partially fluorinated ketone. The term "ketone" is intended to denote a compound incorporating at least one carbonyl group with two carbon atoms attached to the carbon of the carbonyl group. It shall encompass saturated compounds and unsaturated compounds including double and/or triple bonds. The at least partially fluorinated alkyl chain of the ketones can be linear or branched. The term "ketone" shall also encompass compounds with a cyclic carbon backbone. The term "ketone" may comprise additional in-chain hetero-atoms, e.g. at least one heteroatom being part of the carbon backbone and/or being attached to the carbon backbone. More preferably, the at least one compound is a perfluorinated ketone. Examples of suitable perfluorinated ketones include 1,1,1,3,4,4,4-heptafluoro-3-(trifluoromethyl)-butan-2-one; 1,1,1,3,3,4,4,5,5,5-decafluoropentan-2-one; 1,1,1,2,2,4,4,5,5,5-decafluoropentan-3-one, 1,1,1,4,4,5,5,5,-octafluoro-3-bis-(trifluoromethyl)-pentan-2-one; and most preferably heptafluoroisopropyl-trifluoromethyl-ketone.

Also preferably, the at least one compound is a perfluorinated or partially fluorinated ether. The term "ether" is intended to denote a compound incorporating at least one "-C-O-C-" moiety. Especially suitable examples include pentafluoro-ethyl-methyl ether and 2,2,2-trifluoroethyl-trifluoromethyl ether.

Also preferably, the at least one compound is a perfluorinated or partially fluorinated ester, i.e. a compound incorporating at least one "-C(O)O-" moiety. Suitable compounds are known in the art, especially suitable examples include methyl, ethyl, and trifluoromethyl esters of trifluoroacetic acid.

Also preferably, the at least one compound is a perfluorinated or partially fluorinated cyano compound, i.e. a compound incorporating at least one moiety of the structure "-C=N". Preferably, the cyano compound is perfluorinated, more preferably the cyano compound is chosen from the list consisting of perfluorinated methyl, ethyl, isopropyl, propyl, butyl, isobutyl and tertbutyl nitrile.

Also preferably, the at least one compound is a perfluorinated or partially fluorinated hydrocarbon compound. "Hydrocarbon compound" is intended to denote a saturated or unsaturated hydrocarbon, which may in addition to the fluoro substitution also be substituted by other halogen atoms, e.g. Cl, Br, and/or I. Suitable examples include CHF₃, C₂F₄, CF₃CF₂CF₂CF₂I, and CF₂Cl₂.

In a third aspect, the present invention concerns a method for dielectrically insulating an electric active part wherein the active electrical part is arranged in a gas-tight housing comprising the compound according this invention or the gas mixture according to this invention.

The term "active electrical part" has to be understood very broadly. Preferably, it covers any part which is used for the generation, the distribution or the usage of electrical energy provided it comprises a gas-tight housing wherein the dielectric insulating gas provides for the dielectric insulation of parts which bear voltage or current. Preferably, the active electrical parts are medium voltage or high voltage parts. The term "medium voltage" relates to a voltage in the range of 1 kV to 72 kV ; the term "high voltage" refers to a voltage of more than 72 kV. While these are preferred electrical active parts in the frame of the present invention, the parts may also be low voltage parts with a voltage below 1 kV being concerned.

It has to be noted that the electrical active parts of the invention can be "stand alone" parts, or they can be part of an assembly of parts, e.g. of an apparatus. This will now be explained in detail.

The electrical active part can be a switch, for example, a fast acting earthing switch, a disconnector, a load-break switch or a puffer circuit breaker, in particular a medium-voltage circuit breaker (GIS-MV), a generator circuit breaker (GIS-HV), a high voltage circuit breaker, a bus bar, a bushing, a gas-insulated cable, a gas-insulated transmission line, a cable joint, a current transformer, a voltage transformer or a surge arrester.

The electrical active part may also be part of an electrical rotating machine, a generator, a motor, a drive, a semiconducting device, a computing machine, a power electronics device or high frequency parts, for example, antennas or ignition coils.

The method of the invention is especially suited for medium voltage switchgears and high voltage switchgears.

In the electrical active part, the insulating gas is preferably at a pressure of equal to or greater than 0.1 bar (abs.). "Insulating gas" is intended to denote the compounds and/or the mixtures of the present invention in their gaseous state. The insulating gas is at preferably a pressure equal to or lowers than 30 bar (abs). A preferred pressure range is from 1 to 20 bar (abs.).

The partial pressure of the inventive compound of depends, i.a. upon its concentration in the isolating gas. If the dielectric isolating gas consists of the inventive compound its partial pressure is equal to the total pressure and corresponds to the ranges given above. If the dielectric gas includes an inert gas, the partial pressure of the inventive compound is correspondingly lower. A partial pressure of the inventive compound which is equal to or lower than 10 bar (abs) is preferred.

In a preferred embodiment, the insulating gas comprises a compound according to this invention or a mixture according to this invention and an inert gas.

It is also preferred that the compound or the mixture, respectively, is such that under the climate conditions or the temperature in the ambience of the electrical apparatus, under the pressure in the electrical part, essentially no condensation of the components in the dielectric insulating gas occurs. The term "essentially no condensation" denotes that at most 5 % by weight, preferably at most 2 % by weight, of the dielectric insulating gas condenses. For example, the amounts of compound of formula (I) the kind and amount of inert gas are selected such that the partial pressure of compound of formula (I) is lower than the pressure where condensation of compound of formula (I) is observed at -20°C.

In a fourth object, the present invention concerns an apparatus for the generation, distribution and/or usage of electrical energy wherein the apparatus comprises an electrical active part arranged in a gas-tight housing and said gas-tight housing containing an insulating medium comprising or consisting of the compound or the gas mixture according to this invention. Preferably, the apparatus is a switchgear.

In a fifth aspect, the present invention concerns processes for the manufacture of a compound of general formula R1-O-O-R2. R1 and R2 can be different or the same. If R1 and R2 are the same, the compounds can be prepared using the appropriate carbonyl fluorides and molecular fluorine. For example, the perfluorinated diethyl derivative can be produced according to the following equation:

F₂ + 2 CF₃C(O)F → CF₃CF₂OOCF₂CF₃

If R1 and R2 are different and R1 is CF3, the compounds can be prepared using the appropriate carbonyl fluoride, COF₂ and molecular fluorine. For example, the perfluorinated ethyl methyl derivative can be produced according to the following equation:

F₂ + COF₂ + CF₃C(O)F → CF₃OOCF₂CF₃

Alternatively, the compounds of general formula R1-O-O-R2 can be prepared using the appropriate carbonyl fluorides, oxyfluorides and molecular fluorine. In one alternative, the carbonyl fluoride is prepared in situ in a flow reactor, preferably prepared in situ from from F₂ and CO. The mixture of F2 and CO is suitably ignited to prepare the COF₂, e.g. by means of an electrical spark. For example, the perfluorinated diethyl derivative can be produced according to the following equation:

F₂ + CF₃CF₂OF + CF₃C(O)F → CF₃CF₂OOCF₂CF₃

The reactions of this embodiment can be carried out in a static or continuous-flow reactor. Preferably, the reactor is made of a material suitable for reactions involving molecular fluorine, e.g. made of Monel® 400 or nickel. Also preferably, the reaction is carried out in a continuous-flow micro reactor.

Preferably, the reactions of this embodiment are carried out in the presence of a catalyst. Also preferably, in the presence of a metal fluoride. Suitable metal fluorides are AgF, CuF, CuF₂, BaF₂, CsF and NiF₂, specifically AgF.

Preferably, the reaction of this embodiment is carried out at a temperature from -20 to 180 °C, more preferably from 0 to 140 °C, most preferably from 20 to 50 °C and specifically around 25 °C.

Another object of the present invention concerns the use of the compounds or the mixtures of this invention, as herein described, as dielectric insulating gas or as constituent of a dielectric insulating gas. The compounds and the mixtures of the present invention are also useful for other uses, e.g. as fire-extinguishing agent as well as chamber cleaning agent, specifically, for plasma-enhanced chamber cleaning as a replacement for NF₃.

Should the disclosure of any patents, patent applications, and publications which are incorporated herein by reference conflict with the description of the present application to the extent that it may render a term unclear, the present description shall take precedence.

The following examples further explain the invention without intention to limit it.

### Examples

### Example 1a: Manufacture of CF₃-O-O-CF₃

CF₃-O-O-CF₃ is manufactured as described in Example 3 of US-A-2007/0049774.

### Example 1b: Manufacture of CF₃CF₂-O-O-CF₃

An equimolar mixture of carbonyl fluoride (COF₂) and trifluoroacetyl fluoride (CF₃C(O)F) is treated with 1.1 equivalents F₂ in a Monel® 400 continuous flow reactor in the presence of silver fluoride (AgF, 1.0 eq) at room temperature.

### Example 1c: Manufacture of CF₃CF₂-O-O-CF₃

An equimolar mixture of carbonyl fluoride (COF₂) and pentafluoroethyl oxyfluoride (CF₃CF₂OF) is allowed to react in a static reactor made from Monel® 400 in the presence of silver fluoride (AgF , 1.0 eq) at 25 °C for 24 hours.

### Example 1d: Manufacture of CF₃CF₂-O-O-CF₂CF₃

Trifluoroacetyl fluoride (CF₃C(O)F) is treated in a Monel® 400 continuous flow reactor with a 1.1 equivalents F₂ in the presence of silver fluoride (AgF, 1.0 eq) at room temperature.

### Example 1e: Manufacture of CF₃CF₂-O-O-CF₂CF₃

An equimolar mixture of pentafluoroethyl oxyfluoride (CF₃CF₂OF) and trifluoroacetyl fluoride (CF₃C(O)F) is heated to 140 °C in a static reactor made from Monel® 400 in the presence of silver fluoride (AgF, 1.0 eq).

### Example 1f: Manufacture of the mixtures of perfluorinated peroxides like CF₃CF₂-O-O-CF₃, CF₃CF₂-O-O-CF₂-CF₃, CF₃-O-O-CF₃

A flow comprising CO (1 eq) and F₂ (1.05 eq) is ignited. Subsequently, a flow consisting of trifluoroacetyl fluoride (CF₃C(O)F, 1.0 eq) is mixed into the aforementioned flow and the combined flows are directed over a bed of silver fluoride (AgF). The reaction product is trapped in a cold trap at -78 °C.

### Example 2: Manufacture of the gas mixtures

As described in WO9823363, a homogenous gas mixture consisting of CF₃CF₂-O-O-CF₂CF₃ and N₂ in a volume ratio 1:4 is manufactured in an apparatus comprising a static mixer and a compressor.

### Example 3: Provision of an earth cable containing the dielectric insulating gas of example 2

The gas mixture of example 2 is directly fed into an earth cable for high voltage, until a total pressure of 10 bar (abs) in the cable is achieved. *Example 4: A switchgear* containing CF₃CF₂-O-O-CF₂CF₃, *and N₂ in a volume ratio 1:4*

A switchgear is used which contains a switch surrounded by a gas tight metal case. The gas mixture of example 2 is passed into the gas tight metal case via a valve until a pressure of 18 bar (abs) is achieved.

### Example 5: Provision of a gas-insulated transmission line containing the dielectric insulating gas of example 3

The gas mixture of example 2 is directly fed into an earth cable for high voltage, until a total pressure of 10 bar (abs) in the cable is achieved.

## Claims

1. A compound of the general formula R1-O-O-R2 wherein R1 and R2 are independently chosen from the group consisting of perfluorinated or partially fluorinated methyl, ethyl, isopropyl, n-propyl, isobutyl, n-butyl or tert-butyl, with the proviso that if R1 is CF₃, R2 is not CF₃.

2. The compound of claim 1 wherein the R1 and R2 are independently chosen from the group consisting of perfluorinated methyl, ethyl, isopropyl, n-propyl, isobutyl, n-butyl or tert-butyl.

3. The compound of claim 1 or 2 wherein the compound is CF₃CF₂-O-O-CF₂CF₃, (CF₃)₂CF-O-O-CF(CF₃)₂, CF₃-O-O-CF₂CF₃, or CF₃-O-O-CF(CF₃)₂.

4. The compound of any one of claims 1 to 3 wherein the compound has a standard boiling point of less than 20 °C, preferably equal to or lower than 0 °C.

5. A gas mixture of two or more compounds of the general formula R1-O-O-R2 wherein R1 and R2 are independently chosen from the group consisting of perfluorinated or partially fluorinated methyl, ethyl, isopropyl, n-propyl, isobutyl, n-butyl or tert-butyl.

6. The gas mixture of claim 5 wherein the two or more compounds are chosen from the group consisting of CF₃-O-O-CF₃, CF₃CF₂-O-O-CF₂CF₃, (CF₃)₂CF-O-O-CF(CF₃)₂, CF₃-O-O-CF₂CF₃, or CF₃-O-O-CF(CF₃)₂.

7. The gas mixture of claim 5 or 6 further comprising at least one compound selected from an inert gas, a perfluorinated or partially fluorinated ketone, a perfluorinated or partially fluorinated ether, a perfluorinated or partially fluorinated ester, a perfluorinated or partially fluorinated cyano compound, a hydrocarbon compound.

8. The gas mixture of claim 7 wherein the at least one compound is an inert gas selected from the group consisting of air, synthetic air, an air component, N₂, O₂, CO₂, N₂O, He, Ne, Ar, Xe or SF₆; preferably N₂.

9. A method for dielectrically insulating an electric active part wherein the active electrical part is arranged in a gas-tight housing comprising the compound according to any one of claims 1 to 4 or the gas mixture according to any one of claims 5 to 8.

10. A process for the manufacture of a compound of general formula R1-O-O-R1 wherein R1 is chosen from the group consisting of perfluorinated or partially fluorinated methyl, ethyl, isopropyl, n-propyl, isobutyl, n-butyl or tert-butyl; comprising a step of reacting a compound of the general formula R3-C(O)F, wherein R3 is chosen from the group consisting of perfluorinated or partially fluorinated methyl, ethyl, isopropyl and n-propyl, with F₂.

11. The process of claim 10 wherein the step of reacting a compound of the general formula R3-C(O)F is carried out in the presence of a metal fluoride.

12. A process for the manufacture of a compound of general formula R1-O-O-CF₃ wherein R1 is chosen from the group consisting of perfluorinated or partially fluorinated methyl, ethyl, isopropyl, n-propyl, isobutyl, n-butyl or tert-butyl; comprising a step of reacting a compound of the general formula R3-C(O)F, wherein R3 is chosen from the group consisting of perfluorinated or partially fluorinated methyl, ethyl, isopropyl and n-propyl, with COF₂ and F₂.

13. An apparatus for the generation, distribution and/or usage of electrical energy wherein the apparatus comprises an electrical active part arranged in a gas-tight housing and said gas-tight housing containing an insulating medium comprising or consisting of the compound of any one of claims 1 to 4 or the gas mixture of any one of claims 5 or 8.

14. The apparatus of claim 13 wherein the apparatus is a switchgear.
